# FASCICULE DE BREVET EUROPEEN

(11) **EP 0 300 943 B1**
(45) Date de publication et mention de la délivrance du brevet: **21.04.1993**
(21) Numéro de dépôt: 88460014.9
(22) Date de dépôt: 13.07.1988
(51) Int. Cl.: A61B 17/56

(54) **Tige d'enclouage centro-médullaire**
Markraumnagel zur Knochenbruchfixierung
Intramedullary nail

(30) Priorité: 21.07.1987 FR 8710428
(43) Date de publication de la demande: 25.01.1989
(73) Titulaire: LANDOS APPLICATIONS ORTHOPEDIQUES FRANCAISES, F-52000 Chaumont (FR)
(72) Inventeur: Chagneau, Francis, F-35700 Rennes (FR); Levasseur, Michel, F-35510 Cesson-Sevigne (FR); Landanger, Patrick, F-52820 Foulain (FR); Langlais, Frantz, F-35200 Rennes (FR); Rolland, Jean-Jacques, F-22100 Dinan (FR)
(74) Mandataire: Le Faou, Daniel

(56) Documents cités:
- EP-A- 0 145 666
- FR-A- 2 593 057

## Description

La présente invention concerne une tige d'enclouage centro-médullaire, pour le traitement de fractures osseuses.

Pour traiter des fractures osseuses, en particulier des fractures d'os longs tels que tibia ou fémur, il est courant d'utiliser la technique de l'enclouage centro-médullaire. Cette technique consiste à introduire dans un alésage préalablement formé dans le canal centro-médullaire de l'os fracturé, une tige métallique qui va assurer tout d'abord la réduction de la fracture puis le maintien en position correcte des parties disjointes de l'os durant la phase subséquente d'ostéogénèse conduisant à la soudure de ces parties.

Les tiges utilisées à cet effet sont pour certains désignées par l'appellation "clou de Kuntscher".

Il s'agit d'un tube métallique, généralement en acier inoxydable, qui est fendu longitudinalement ; il présente donc une section en forme de C. La fente, qui mesure quelques millimètres de largeur, confère au clou une certaine élasticité en direction radiale, les bords de la fente pouvant se rapprocher ou s'écarter élastiquement l'un de l'autre. Cette élasticité permet d'obtenir un contact intime entre la paroi externe du clou et la paroi alésée de l'os, ce qui est nécessaire pour un bon maintien des parties disjointes.

La présence de cette fente n'affecte pratiquement pas la résistance à la flexion de la tige. Par contre, la présence de la fente diminue très sensiblement la résistance de la tige à la torsion. En effet, si un tube est fendu longitudinalement, il se déforme beaucoup plus facilement sous l'action d'un couple de torsion que s'il n'était pas fendu, les deux bords de la fente ayant tendance à se rapprocher en décrivant une hélice.

On comprendra aisément qu'une souplesse excessive en torsion n'est pas souhaitable car elle autorise des déplacements relatifs des parties disjointes, ce qui contrarie et retarde la consolidation du tissu osseux au niveau de la fracture.

C'est pourquoi, pour obtenir une résistance en torsion acceptable, les fabricants ont été obligés jusqu'ici de prévoir pour ce type de clou une épaisseur de paroi relativement importante, voisine de 2 mm et plus, qui ne serait pas justifiée autrement. Malheureusement, l'épaisseur excessive de la paroi diminue corrélativement l'élasticité radiale du clou, et les exigences contradictoires relatives à la rigidité en torsion et à l'élasticité radiale conduisent à un compromis qui n'est pas toujours parfaitement satisfaisant. D'autre part, une épaisseur de paroi importante conduit à un poids élevé, ce qui constitue un inconvénient pour le patient, dans la mesure où ce genre de tige doit être conservée dans l'os durant des périodes longues.

On connaît par ailleurs, par le document EP-A-0 145 666, un clou intra-médullaire qui comporte une fente longitudinale s'étendant sur toute la longueur du clou. Cette caractéristique est destinée à éviter les concentrations de contraintes qui apparaissent sur les clous traditionnels, à fente incomplète, dans la zone d'extrémité de cette fente, afin d'assurer une élasticité homogène du clou sur toute sa longueur. Toutefois, pour éviter des difficultés au moment de la pose et de l'enlèvement du clou, dans la partie proximale de celui-ci (intérieurement taraudée), la fente a une largeur très faible par rapport à la fente traditionnelle. Dans cette partie est prévue une forme en chicane, en queue d'aronde, dont le rôle est de maintenir les deux bords de la fente rapprochés et d'empêcher l'expansion de cette partie du clou.

Le clou connu, qui possède sur la plus grande partie de sa longueur une fente traditionnelle, ne présente pas une meilleure résistance à la torsion que les clous classiques.

L'invention vise à résoudre ces différents problèmes en proposant une tige d'enclouage centro-médullaire du genre mentionné qui, tout en ayant une bonne élasticité en direction radiale, possède une résistance accrue à la torsion.

Ces différents résultats sont obtenus conformément à l'invention, grâce au fait que la tige comporte des moyens de verrouillage aptes à empêcher ou limiter le déplacement relatif des deux bords de la fente suivant la direction longitudinale, tout en autorisant leur déplacement relatif en direction transversale.

Dans un premier mode de réalisation de l'invention, les moyens de verrouillage comprennent une série de languettes transversales qui sont solidaires de l'un des bords de la fente et pénètrent dans des découpes de forme complémentaire formées dans l'autre bord, ce qui donne à la fente une forme en chicane.

La coopération des bords transversaux de la fente avec les bords correspondants des découpes permet d'absorber les efforts longitudinaux qui se développent lorsque la tige est soumise à un couple de torsion, empêchant l'enroulement en hélice des deux bords de la fente.

Dans un second mode de réalisation de l'invention, les moyens de verrouillage comprennent une série de languettes transversales qui sont solidaires de l'un des bords de la fente et pénètrent dans des fenêtres ménagées dans des languettes solidaires de l'autre bord ; ces languettes sont de préférence repliées à l'intérieur de la tige tubulaire, de manière à ne pas dépasser à l'extérieur du contour de la tige.

D'autres caractéristiques et avantages apparaîtront de la description et des dessins annexés qui en présentent des modes de réalisation préférentiels.

Sur les dessins :
- la figure 1 est une vue en coupe longitudinale d'une tige conforme à l'invention ;
- la figure 2 est une vue de droite du clou de la figure 1 ;
- les figures 3 et 4 sont des sections, à échelle légèrement agrandie, du clou des figures 1 et 2 coupé par les plans transversaux III-III et IV-IV respectivement ;
- la figure 5 est une vue de détail à grande échelle montrant la coopération des moyens de verrouillage languette/découpe dont est muni le clou des figures 1 et 2 ;
- la figure 6 représente à grande échelle, en coupe transversale, une variante des moyens de verrouillage ;
- la figure 7 est une section partielle des moyens de verrouillage de la figure 6, coupés par le plan longitudinal VII.

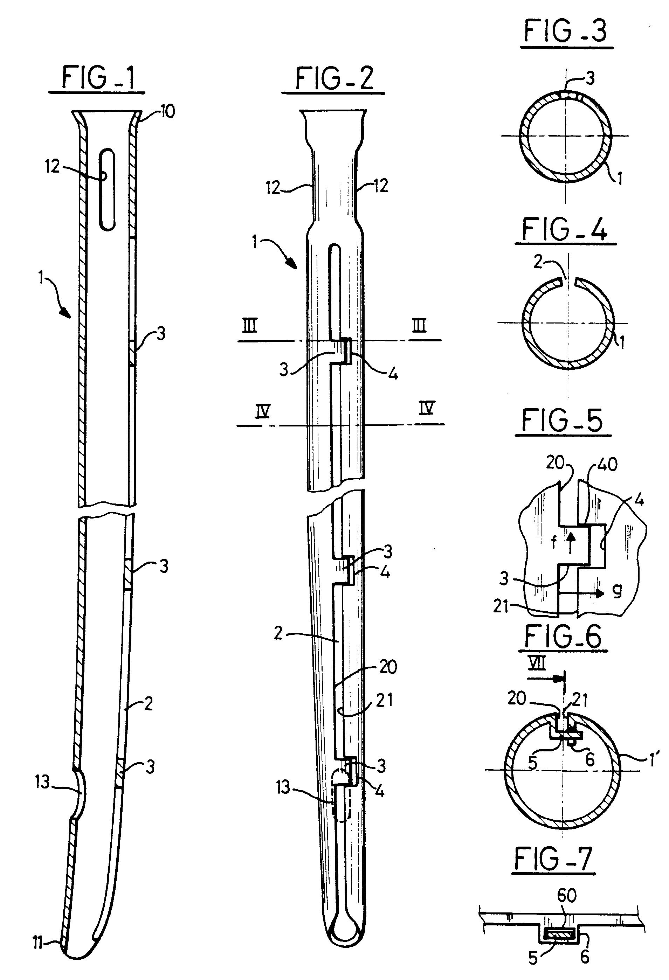

La tige d'enclouage centro-médullaire représentée sur les figures 1 et 2 a la forme générale d'un clou métallique tubulaire. Sa longueur et son diamètre dépendent de l'utilisation à laquelle il est destiné ; à titre d'exemple, pour l'enclouage d'un fémur, la longueur du clou se trouvera dans une plage comprise entre 36 et 48 cm, et son diamètre moyen dans une plage comprise entre 12 et 16 mm ; pour un tibia, ces plages seront respectivement de 28 à 38 cm et de 11 à 15 mm.

De manière traditionnelle, ce clou 1 présente une extrémité proximale 10 légèrement évasée (en forme de collerette), et une extrémité distale 11 légèrement effilée, recourbée et arrondie ; les extrémités proximale et distale présentent des ouvertures 12, respectivement 13, qui autorisent le passage de moyens de verrouillage appropriés, par exemple de vis ou d'autres moyens de fixation, de manière à permettre une solidarisation parfaite de la tige avec chacune des parties de l'os fracturé.

La tige 1 présente une fente 2, qui s'étend longitudinalement sur la plus grande partie de sa longueur. Contrairement au clou selon le document EP-A-0 145 666 précité, cette fente est incomplète, l'extrémité proximale 10 n'étant pas fendue. La largeur de la fente est de quelques millimètres, par exemple de 2 mm.

Comme expliqué plus haut, la présence de la fente 2 donne à la tige une certaine élasticité dans le sens radial, le rapprochement élastique des deux bords 20 et 21 de la fente étant possible, ce qui permet d'assurer un contact intime de la tige avec la paroi de l'os lors de sa mise en place.

Conformément à l'invention, l'un des bords 20 de la fente 2 est découpé de manière à présenter une série de languettes 3, de forme carrée ou rectangulaire, qui traversent la fente 2 et pénètrent dans des découpes 4 ménagées dans l'autre bord 21.

Le nombre de languettes 3 (et de découpes 4) est par exemple compris entre deux et six, ces organes de verrouillage étant espacés - régulièrement ou non - sur toute la longueur de la fente 2.

La fente 2 a par conséquent une forme en chicane.

Comme on le comprend directement à l'observation de la figure 5, la présence des languettes 3 ne va pas contrarier le mouvement transversal relatif des bords 20 et 21 (flèche g) ; on notera en effet que la languette 3 n'est pas en contact avec le fond de la découpe 4, un jeu étant conservé à ce niveau.

Si on soumet la tige à un effort de torsion, c'est-à-dire si on tend à la vriller autour de son axe longitudinal, les deux bords 20 et 21 ont tendance à se déplacer longitudinalement l'un par rapport à l'autre, tout en se rapprochant et prenant la forme d'une hélice. Cependant, un tel déplacement relatif n'est pas possible, puisque le bord transversal de la languette vient prendre appui contre l'un ou l'autre des bords latéraux 40 de la découpe correspondante ; à la figure 5, on a représenté par la flèche (f) l'effort longitudinal résultant de la torsion, qui va être absorbé par l'appui de la languette 3 contre le bord de découpe 40.

Ainsi, tout au long de la fente, des moyens d'arrêt empêchent le déplacement relatif des bords 20 et 21 et leur enroulement en hélice, si bien que la déformation en torsion de la tige est considérablement réduite, comparable à celle que prendrait un tube fermé sous un effort analogue.

Dans la variante des figures 6 et 7, le bord 20 est pourvu d'une série de languettes 5 analogues aux languettes 3 qui viennent d'être décrites, les languettes 5 étant cependant repliées vers l'intérieur du clou tubulaire 1. L'autre bord 21 est pourvu de languettes 6 rabattues également à l'intérieur du tube, ces languettes 6 présentant une ouverture ou fenêtre 60 adaptée pour recevoir, avec possibilité de glissement, une languette 5 ; l'effet de ces moyens de verrouillage 5, 6 est le même que celui des moyens 3 et 4 de la forme de réalisation précédente ; ils autorisent le rapprochement et l'écartement mutuels des bords 20 et 21, tout en empêchant le déplacement relatif de ces bords dans le sens longitudinal.

Bien qu'il soit préférable de prévoir plusieurs moyens de verrouillage répartis le long de la tige 1, on pourrait concevoir, sans sortir du cadre de l'invention, l'existence d'un seul moyen de verrouillage, par exemple une languette unique 3 coopérant avec une découpe 4, ce moyen de verrouillage étant situé à peu près au milieu de la tige. De plus, la forme de la (ou des) languette(s) n'est pas obligatoirement quadrangulaire, différentes formes pouvant être prévues qui permettent d'empêcher ou de limiter le déplacement relatif des deux bords 20 et 21 dans le sens longitudinal, suivant f. Ces formes pourraient en outre être conçues de manière à verrouiller plus ou moins le déplacement longitudinal en fonction de la compression radiale de la tige.

Grâce à l'invention, il est possible de fabriquer ce genre de tige avec une épaisseur de paroi relativement mince (1 à 1,5 mm), ce qui allège considérablement la tige.

Le même principe peut être utilisé pour la fabrication de clous centro-médullaires spéciaux, dont les extrémités possèdent des moyens d'ancrage escamotables, comme par exemple celui qui fait l'objet du brevet français n^{o} 2 567 016.

## Revendications

1. Tige d'enclouage centro-médullaire pour le traitement de fractures osseuses, qui affecte la forme générale d'un tube fendu longitudinalement, caractérisée par le fait qu'elle comporte des moyens de verrouillage (3-4 ; 5-6) aptes à empêcher ou limiter le déplacement relatif des deux bords (20-21) de la fente (2) suivant la direction longitudinale, tout en autorisant leur déplacement relatif en direction transversale.

2. Tige selon la revendication 1, caractérisée par le fait que lesdits moyens de verrouillage comprennent une série de languettes transversales (3) qui sont solidaires de l'un des bords (20) de la fente (2) et pénètrent dans des découpes (4) de forme complémentaire formées dans l'autre bord (21), ce qui donne à la fente (2) une forme en chicane.

3. Tige selon la revendication 1, caractérisée par le fait que lesdits moyens de verrouillage comprennent une série de languettes transversales (5) qui sont solidaires de l'un des bords (20) de la fente (2) et pénètrent dans des fenêtres (60) ménagées dans des languettes (6) solidaires de l'autre bord (21).

4. Tige selon la revendication 3, caractérisée par le fait que lesdites languettes (5-6) sont repliées à l'intérieur de la tige tubulaire (1).

## Claims

1. A centro-medullary nailing rod for treating bone fractures, said rod being generally in the form of a longitudinally split tube, characterized in that it includes locking means (3-4 ; 5-6) suitable for preventing or limiting relative displacement between the two edges (20-21) of the slot (2) in the longitudinal direction, while allowing relative displacement thereof in the transverse direction.

2. A rod according to claim 1, characterized in that said locking means comprise a series of transverse tongues (3) fixed to one of the edges (20) of the slot (2) and penetrating into cut-outs (4) of complementary shape formed in the other edge (21), thereby imparting an intermeshing shape to the slot (2).

3. A rod according to claim 1, characterized in that said locking means comprise a series of transverse tongues (5) which are fixed to one of the edges (20) of the slot (2) and which penetrate into windows (60) provided in tongues (6) fixed to the other edge (21).

4. A rod according to claim 3, characterized in that said tongues (5-6) are folded into the tubular rod (1).

## Patentansprüche

1. Markraumnagel für die Behandlung von Knochenbrüchen, der die allgemeine Form eines längsgeschlitzten Rohres aufweist, dadurch **gekennzeichnet,**
daß er Verriegelungsmittel (3-4; 5-6) umfaßt, die geeignet sind, die relative Bewegung der beiden Ränder (20-21) des Schlitzes (2) in Längsrichtung zu verhindern, jedoch ihre relative Bewegung in Querrichtung zuzulassen.

2. Nagel nach Anspruch 1, dadurch gekennzeichnet, daß die genannten Verriegelungsmittel eine Reihe von Querlaschen (3) aufweisen, die fest verbunden sind mit einem der Ränder (20) des Schlitzes (2) und in die komplementär geformten Aussparungen (4) eindringen, die in dem anderen Rand ausgebildet sind, was dem Schlitz (2) eine Zickzack- oder Mäanderform verleiht.

3. Nagel nach Anspruch 1, dadurch gekennzeichnet, daß die Verriegelungsmittel eine Reihe von Querlaschen (5) enthalten, die fest mit einem der Ränder (20) des Schlitzes (2) verbunden sind und in Schlitzlöcher (60) eindringen, die in den mit dem anderen Rand (21) fest verbundenen Laschen (6) ausgespart sind.

4. Nagel nach Anspruch 3, dadurch gekennzeichnet, daß die genannten Laschen (5-6) im Innern des rohrförmigen Nagels (1) umgebogen sind.
